(19) Europäisches Patentamt
European Patent Office
Office européen des brevets

(11) Veröffentlichungsnummer: **0 103 277**
**A1**

# (12) EUROPÄISCHE PATENTANMELDUNG

(21) Anmeldenummer: **83108871.1**

(22) Anmeldetag: **08.09.83**

(51) Int. Cl.³: **C 07 D 307/91**, C 07 D 405/12, C 07 D 413/12, A 01 N 43/12

(30) Priorität: **15.09.82 DE 3234176**

(43) Veröffentlichungstag der Anmeldung: **21.03.84 Patentblatt 84/12**

(84) Benannte Vertragsstaaten: **AT BE CH DE FR GB IT LI NL SE**

(71) Anmelder: **BASF Aktiengesellschaft, Carl-Bosch-Strasse 38, D-6700 Ludwigshafen (DE)**

(72) Erfinder: **Meyer, Norbert, Dr., Stahlbuehlring 155a, D-6802 Ladenburg (DE)**
Erfinder: **Rentzea, Costin, Dr., Richard-Kuhn-Strasse 1-3, D-6900 Heidelberg (DE)**
Erfinder: **Pommer, Ernst-Heinrich, Dr., Berliner Platz 7, D-6703 Limburgerhof (DE)**
Erfinder: **Ammermann, Eberhard, Dr., Sachsenstrasse 3, D-6700 Ludwigshafen (DE)**

(54) **Quartäre Ammoniumsalze von Benzofuranderivaten und diese enthaltende Fungizide.**

(57) Quartäre Ammoniumsalze der Formel

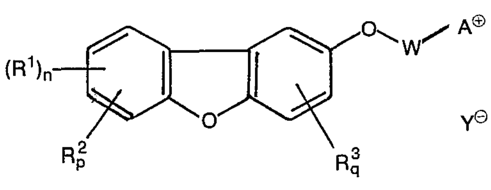

$Y^{\ominus}$ (I)

in der $R^1$, $R^2$ und $R^3$ gleich oder verschieden sind und Halogen, eine gegebenenfalls substituierte Alkyl- oder Alkoxygruppe, Cyan oder Nitro, n, p und q die Zahlen 0, 1, 2 oder 3, W eine substituierte Alkylenkette, A die Gruppe $-NR^4R^5R^6$, in der $R^4$, $R^5$ und $R^6$ einen Alkyl-, Alkenyl- oder Alkinylrest, einen Cycloalkylrest oder einen Cycloalkenylrest, wobei diese Reste substituiert sein können, oder einen gegebenenfalls substituierten Aralkyl- oder Aryloxyalkylrest bedeuten oder $R^5$ zusammen mit $R^6$ Teil eines gegebenenfalls substituierten heterocyclischen Ringes ist, und Y das Anion einer nicht phytotoxischen Säure HY bedeutet und diese enthaltende Fungizide.

EP 0 103 277 A1

BASF Aktiengesellschaft O.Z. 0050/36147

Quartäre Ammoniumsalze von Benzofuranderivaten und diese enthaltende Fungizide

Die vorliegende Erfindung betrifft neue quartäre Ammoniumsalze, Verfahren zu ihrer Herstellung und fungizide Mittel, die diese Verbindungen als Wirkstoffe enthalten.

Es ist bekannt, N-Trichlormethylthiotetrahydrophthalimid als Fungizid zu verwenden (Chem.Week, June 21, Seite 46, 1972).

Es wurde gefunden, daß quartäre Ammoniumsalze von Benzofuranderivaten der Formel

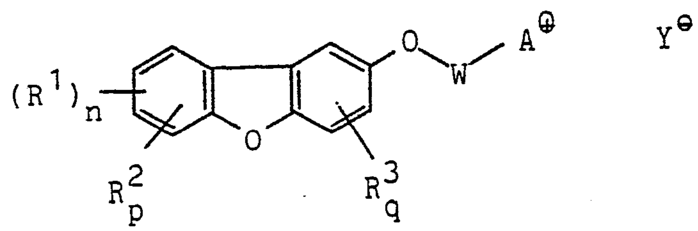

(I)

in der $R^1$, $R^2$ und $R^3$ gleich oder verschieden sind und Halogen, eine gegebenenfalls halogensubstituierte Alkyl- oder Alkoxygruppe mit 1 bis 4 Kohlenstoffatomen, Cyan oder Nitro,
n, p und q die Zahlen 0, 1, 2 oder 3,
W eine durch eine oder mehrere Alkylgruppen mit jeweils 1 bis 3 C-Atomen substituierte Alkylenkette mit 2 bis 5 C-Atomen,
A die Gruppe $-NR^4R^5R^6$, in der $R^4$, $R^5$ und $R^6$ gleich oder verschieden sind und unabhängig voneinander einen Alkyl-, Alkenyl- oder Alkinylrest jeweils mit bis zu 6 Kohlenstoffatomen, einen Cycloalkylrest mit 3 bis 12 Kohlenstoffatomen oder einen Cycloalkenylrest mit 4 bis 7 Kohlenstoffatomen, wobei diese acyclischen und cyclischen Reste durch Halogen, Cyan, Alkoxy mit 1 bis 4 Kohlenstoffatomen oder Dialkylamino mit 1 bis 4 Kohlenstoffatomen in jeder Alkylgruppe substituiert sein können, oder einen gegebenenfalls durch Fluor, Chlor, Brom, Alkyl-, Alkenyl- oder Alkoxyrest jeweils mit bis zu 4 Kohlenstoffatomen, Trifluormethyl, Nitro, Cyan oder Alkoxycarbonyl mit 2 bis 5 Kohlenstoffatomen substituierten Aralkyl- oder Aryloxyalkylrest bedeuten oder $R^5$ zusammen mit $R^6$ Teil eines gegebenenfalls durch eine oder mehrere Alkylgruppen mit 1 bis 4 Kohlenstoffatomen substituierten 5-, 6- oder 7-gliedrigen heterocyclischen Ringes mit 1 bis 3 Heteroatomen ist, und
Y das Anion einer nicht phytotoxischen Säure HY bedeutet, fungizid gut wirksam sind.

Sws/Kl

Als Bedeutungen für $R^1$, $R^2$ und $R^3$ in Formel I kommen Halogen, wie Fluor, Chlor, Brom, Jod, Nitro, Cyano oder gegebenenfalls durch Halogen substituierte Alkyl- oder Alkoxyreste mit 1 bis 4 Kohlenstoffatomen, wie Methyl, Ethyl, Isopropyl, tert.-Butyl, Methoxy, Ethoxy, Trifluormethyl, Difluormethyl, Tetrafluorethoxy, in Betracht.

A in Formel I bedeutet die Gruppe $-NR^4R^5R^6$. $R^4$, $R^5$ und $R^6$ stehen darin für Alkyl-, Alkenyl- oder Alkinylgruppen mit bis zu 6 Kohlenstoffatomen, vorzugsweise mit bis zu 4 Kohlenstoffatomen, die durch Halogen, wie Fluor, Chlor, Brom, Alkoxy mit 1 bis 4 Kohlenstoffatomen, Cyano oder Dialkylamino mit 1 bis 4 Kohlenstoffatomen in jeder Alkylgruppe substituiert sein können, beispielsweise Methyl, Ethyl, n-Propyl, Isopropyl, n-Butyl, Isobutyl, n-Pentyl, Isopentyl, n-Hexyl, Allyl, 2-Butenyl, 4-Chlor-2-butenyl, Propargyl, Trifluormethyl, 2-Chlorethyl und 2-Bromethyl, oder für Cycloalkylreste mit 3 bis 12 Kohlenstoffatomen oder Cycloalkenylreste mit 4 bis 7 Kohlenstoffatomen, die durch Halogen, wie Fluor, Chlor, Brom, Alkoxy mit 1 bis 4 Kohlenstoffatomen, Cyano oder Dialkylamino mit 1 bis 4 Kohlenstoffatomen in jeder Alkylgruppe substituiert sein können, beispielsweise Cyclohexyl, 2-Dimethylaminocyclohexyl und Cyclododecyl, oder für einen gegebenenfalls durch Fluor, Chlor, Brom, Alkyl-, Alkenyl- oder Alkoxyrest mit bis zu 4 Kohlenstoffatomen, Trifluormethyl, Nitro, Cyano oder Alkoxycarbonyl mit bis zu 5 Kohlenstoffatomen substituierte Aralkylreste, wie Benzyl, Phenethyl, Naphthylmethyl, beispielsweise 2-Fluorbenzyl, 4-Fluorbenzyl, 2-Chlorbenzyl, 4-Chlorbenzyl, 3-Brombenzyl, 4-Brombenzyl, 3-Trifluormethylbenzyl, 4-Trifluormethylbenzyl, 2-Methylbenzyl, 3-Methylbenzyl, 4-Methylbenzyl, 4-tert.-Butylbenzyl, 4-Nitrobenzyl, 2,4-Dichlorbenzyl, 3,4-Dichlorbenzyl, 2,4-Dimethylbenzyl, 3,4-Dimethylbenzyl, 4-Methoxybenzyl und 1-Naphthylmethyl oder Aryloxyalkylreste mit 2 bis 5 C-Atomen im Alkylrest, wobei das Aryl beispielsweise 1- und 2-Naphthyl, Biphenyl, Phenyl, 4-Fluorphenyl, 3- und 4-Chlorphenyl, 4-Bromphenyl, 3,4-Dichlorphenyl, 2,4-Dichlorphenyl, 2,6-Dichlorphenyl, 2-Methyl-4-chlorphenyl, 4-Methoxyphenyl, 4-Ethoxyphenyl, 3-Methylphenyl, 4-Methylphenyl, 4-Isopropylphenyl, 4-tert.-Butylphenyl, 4-Nitrophenyl, 4-Trifluormethylphenyl, 2,4-Dimethylphenyl oder 4-Cyanphenyl ist.

$R^5$ kann auch zusammen mit $R^6$ eine Alkylengruppe bilden, die zusammen mit dem quartären Stickstoffatom, dessen Substituenten $R^5$ und $R^6$ sind, einen gegebenenfalls durch eine oder mehrere Alkylgruppen mit 1 bis 4 Kohlenstoffatomen, wie Methyl, Ethyl oder t-But l substituierten 5-, 6- oder 7-gliedrigen heterocyclischen Ring mit 1 bis 3 Heteroatomen im Ring bildet, beispielsweise einen Pyrrolidinium-, Piperidinium-, Hexamethylen-

iminium-, Morpholinium- oder Thiomorpholiniumrest, der am quartären Stickstoff durch $R^4$ substituiert ist.

Da die Wirkung der neuen Verbindungen der Formel I auf das Kation zurückzuführen ist, kann das Anion $Y^{\ominus}$ aus nicht phytotoxischen Säuren beliebig gewählt werden. Y bedeutet beispielsweise Methylsulfonat, p-Dodecylbenzolsulfonat, Sulfat, Methosulfat, Nitrat, Phosphat, Jodid und insbesondere Chlorid oder Bromid.

Bevorzugt werden Verbindungen der Formel I, in der $R^1$ Halogen, wie Fluor, Chlor, Brom oder Trifluormethyl, n die Zahl 1 und p und q die Zahl 0 bedeuten.

In der Gruppe $-NR^4R^5R^6$ werden $R^4$, $R^5$, $R^6$ bevorzugt, die gleich oder verschieden sind und unabhängig voneinander unverzweigte oder verzweigte, gegebenenfalls durch Halogen substituierte Alkylreste mit 1 bis 5 Kohlenstoffatomen, wie Methyl, Ethyl, n-Propyl, Isopropyl, n-Butyl, Isobutyl, n-Pentyl, Isopentyl, 2-Chlorethyl, 3-Chlorpropyl, 2-Bromethyl, 3-Brompropyl, 4-Brombutyl, Alkenylreste mit 3 oder 4 Kohlenstoffatomen, wie Allyl, 2-Butenyl, oder Cycloalkylreste mit 3 bis 6 Kohlenstoffatomen, wie Cyclopropyl, Cyclohexyl, bedeuten.

In einer weiteren Gruppe bevorzugter Verbindungen bedeutet $R^4$ gegebenenfalls durch Fluor, Chlor, Brom, Trifluormethyl, Nitro oder Alkyl mit 1 bis 4 Kohlenstoffatomen substituiertes Benzyl, wie Benzyl, 2-Fluorbenzyl, 4-Fluorbenzyl, 2-Chlorbenzyl, 4-Chlorbenzyl, 3-Brombenzyl, 4-Brombenzyl, 3-Trifluormethylbenzyl, 4-Trifluormethylbenzyl, 2-Methylbenzyl, 3-Methylbenzyl, 4-Methylbenzyl, 4-tert.-Butylbenzyl, 4-Nitrobenzyl, 2,4-Dichlorbenzyl, 3,4-Nitrobenzyl, 2,4-Dimethylbenzyl oder 3,4-Dimethylbenzyl, und $R^5$ ist mit $R^6$ Teil eines gegebenenfalls durch eine oder mehrere Alkylgruppen mit 1 bis 4 Kohlenstoffatomen substituierten 5-, 6- oder 7-gliedrigen heterocyclischen Ringes mit 1 bis 3 Heteroatomen, beispielsweise eines Pyrrolidinium-, Piperidinium-, Hexamethyleniminium-, Morpholinium-, Thiomorpholinium-, 2,6-Dimethylmorpholinium-, 2,6-Dimethyl-thiomorpholinium-, 2-Ethyl-piperidinium-, 4-Methyl-piperidinium-, 4-tert.-Butyl-piperidiniumrestes.

Man erhält die quartären Ammoniumsalze der Formel I beispielsweise durch Umsetzung von

a) einer Verbindung der Formel

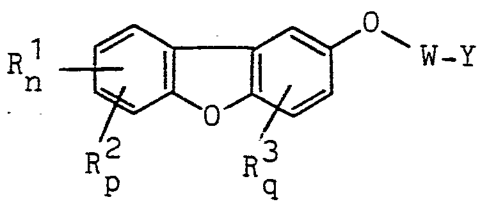

(II)

in der $R^1$, $R^2$, $R^3$, W, Y, n, p und q die oben angegebenen Bedeutungen haben, mit einem tertiären Amin der Formel A ($NR^4R^5R^6$) entsprechend den oben angegebenen Bedeutungen oder

b) einer Verbindung der Formel

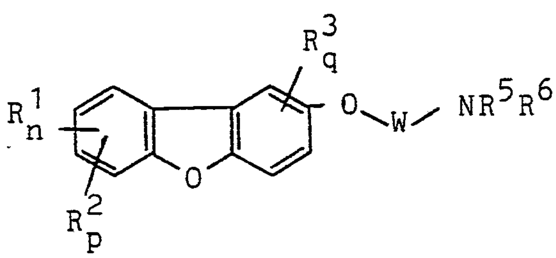

(III),

in der $R^1$, $R^2$, $R^3$, $R^5$, $R^6$, W, n, p und q die oben angegebenen Bedeutungen haben, mit einer Verbindung der Formel

$$R^4 - Y \qquad (IV),$$

in der $R^4$ und Y die oben angegebenen Bedeutungen haben.

Die Umsetzung b) wird bevorzugt.

Die Reaktionen a) und b) werden gegebenenfalls in Gegenwart eines Lösungs- oder Verdünnungsmittels bei einer Temperatur im Bereich zwischen 20 und 150°C, vorzugsweise zwischen 30 und 140°C, durchgeführt. Der Ausgangsstoff der Formel II wird zweckmäßig bei der Umsetzung a) mit dem 2- bis 10-fachen molaren Überschuß des Amins der Formel A umgesetzt.

Als bevorzugte, gegenüber den Reaktionsteilnehmern inerte Lösungs- oder Verdünnungsmittel können beispielsweise aliphatische oder aromatische, gegebenenfalls halogenierte Kohlenwasserstoffe, wie Pentan, Cyclohexan, Benzol, Toluol, Xylol, Chlorbenzol, Dichlorbenzole, aliphatische Ketone, wie Aceton, Methylethylketon, Diethylketon oder Cyclopentanon, Ether, wie Diethylether, Dimethoxyethan, Tetrahydrofuran oder Dioxan, Ester, wie Essigsäureethylester, Nitrile, wie Acetonitril, Amide, wie Dimethylformamid, Dimethylacetamid und N-Methylpyrrolidon, oder Gemische dieser Lösungsmittel verwendet werden.

Die Ausgangsstoffe der Formel II können leicht nach bekannten Verfahren hergestellt werden, beispielsweise durch Veretherung von Dibenzofuran-3--olen mit aliphatischen Dihalogeniden, bevorzugt in siedendem Methylethylketon, Diethylketon oder Cyclopentanon in Gegenwart von mindestens äquivalenten Mengen Natrium- oder Kaliumcarbonat (Houben-Weyl-Müller, Methoden der Organischen Chemie, Band 6/3, Seiten 54 bis 59, Georg Thieme Verlag, Stuttgart, 1965).

Alternativ können Dibenzofuran-3-oxyalkanole mit Thionylchlorid oder Phosphortribromid zu Verbindungen der Formel II umgesetzt werden (Rec.trav.Chim. 76, 129 bis 146 (1957)). Dibenzofuran-3-oxyalkanole erhält man durch Umsetzung von Dibenzofuran-3-olen mit aliphatischen Oxiranen oder aliphatischen Carbonaten nach bekannten Methoden.

Weiterhin lassen sich entsprechend Literaturangaben (J. Med.Chem. 1974, Vol. 17, Seite 108) hergestellte alpha-(Dibenzofuran-3-oxy)-carbonsäureester katalytisch oder mit komplexen Hydriden zu Dibenzofuran-3-oxyalkanolen reduzieren.

Als alkylsubstituierte Alkylketten W kommen beispielsweise in Betracht $CH(CH_3)-CH_2$, $CH_2CH(CH_3)$, $CH(C_2H_5)-CH_2$, $CH_2CH(CH_3)-CH_2$, $CH_2CH_2CH(CH_3)CH_2CH_2$, $CH_2CH_2CH(CH_3)$, $CH_2CH(CH_3)CH_2CH(CH_3)CH_2$, $CH(CH_3)CH_2CH(CH_3)$, $CH(C_3H_7)CH_2$.

Als tertiäre Amine der Formel A können z.B. Trimethylamin, Triethylamin, Tripropylamin, Methyl-diethylamin, Methyl-dipropylamin, Tributylamin, Tripentylamin, 1,2-Bis-(dimethylamino)-cyclohexan, N,N-Dimethyl-N-cyclododecylamin, N,N-Dimethyl-N-cyclohexylamin, N,N-Dimethyl-N-benzylamin, N-Methylpyrrolidin, N-Ethylpyrrolidin, N-Allylpyrrolidin, N-Propylpyrrolidin, N-Butylpyrrolidin, N-Methylpiperidin, N-2-Dimethylpiperidin, N-3-Dimethylpiperidin, N-4-Dimethylpiperidin, N-2,4-Trimethylpiperidin, N-3,5-Trimethylpiperidin, N-2,5-Trimethylpiperidin, N-Methyl-2-ethylpiperidin, N-Ethylpiperidin, N-Methyl-3-hydroxymethylpiperidin, N-Methylmorpholin, N-Ethylmorpholin, N-2,6-Trimethylmorpholin, N-Ethyl-2,6--dimethylmorpholin, N-Methyl-hexamethylenimin, N-Propyl-hexamethylenimin, eingesetzt werden.

Die tertiären Amine der Formel III lassen sich nach bekannten Verfahren herstellen, beispielsweise nach dem Schema

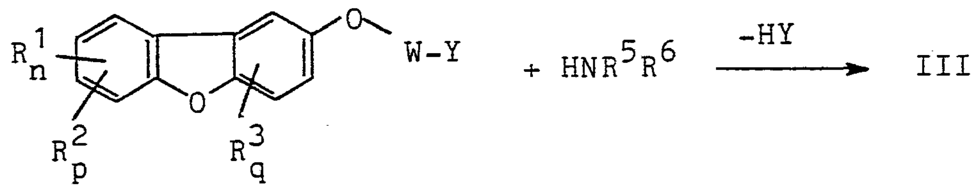

durch Alkylierung sekundärer Amine der Formel $HNR^5R^6$, in der $R^5$ und $R^6$ die obengenannten Bedeutungen haben, mit den Verbindungen der Formel II. Die Reaktionsbedingungen entsprechen hierbei denen der Umsetzung a).

Die bei der Umsetzung entstehende Säure HY kann leicht entfernt werden, z.B. durch Behandeln des Reaktionsgemisches mit wäßrigen Lösungen von Alkalihydroxiden.

Als Amine der Formel $HNR^5R^6$ kommen beispielsweise Dimethylamin, Dipropylamin, Diisopropylamin, Diallylamin, Dibutylamin, Diisobutylamin, N-Methylbenzylamin, Pyrrolidin, Piperidin, 2-Methylpiperidin, 3-Methylpiperidin, 4-Methylpiperidin, 2,4-Dimethylpiperidin, 3,5-Dimethylpiperidin, 2,6--Dimethylpiperidin, 2-Ethylpiperidin, 4-tert.-Butylpiperidin, N-Ethylbenzylamin, N-Butylbenzylamin, Morpholin, 2-Methylmorpholin, 3-Methylmorpholin, 2,6-Dimethylmorpholin, 2,5-Dimethylmorpholin, 2,6-Dimethylthiomorpholin oder Hexamethylenimin in Betracht.

Als Verbindungen der Formel IV können beispielsweise Methylchlorid, Methylbromid, Methyljodid, Dimethylsulfat, Ethylchlorid, Ethylbromid, Ethyljodid, Diethylsulfat, Dibrommethan, 2-Methoxyethylbromid, Propylbromid, Propyljodid, 1-Chlor-3-brompropan, 1,3-Dibrompropan, Isopropylbromid, Allylchlorid, Allylbromid, n-Butylbromid, Butylchlorid, 1,4-Dibrombutan, 1,4-Dichlorbutan, Propargylchlorid, Propargylbromid, 1-Brom-2--buten, 1,4-Dichlor-2-buten, Benzylchlorid, Benzylbromid, 2-Fluorbenzylchlorid, 3-Fluorbenzylbromid, 4-Fluorbenzylbromid, 2-Chlorbenzylchlorid, 3-Chlorbenzylbromid, 4-Chlorbenzylbromid, 4-Brombenzylbromid, 2,4-Dichlorbenzylchlorid, 3,4-Dichlorbenzylchlorid, 4-Methylbenzylbromid, 2,4-Dimethylbenzylbromid, 3-Trifluormethylbenzylchlorid, 4-Trifluormethylbenzylbromid, 4-Nitrobenzylchlorid, 4-Nitrobenzylbromid, 4-tert.-Butylbenzylbromid, 3,4,5-Trimethoxybenzylchlorid, 4-Cyanbenzylchlorid und 1-Chlormethylnaphthalin eingesetzt werden.

Für die Herstellung von Ausgangsverbindungen der Formel II können beispielsweise die folgenden Dibenzofuran-3-ole eingesetzt werden:

Dibenzofuran-3-ol, 7-Fluor-dibenzofuran-3-ol, 7-Chlordibenzofuran-3-ol, 2,4,7-Trichlor-dibenzofuran-3-ol, 5-Chlordibenzofuran-3-ol, 6-Chlor-dibenzofuran-3-ol, 8-Chlor-dibenzofuran-3-ol, 5,7-Dichlorbenzofuran-3-ol, 6,7-Dichlor-dibenzofuran-3-ol, 7,8-Dichlordibenzofuran-3-ol, 7-Bromdibenzofuran-3-ol, 7-Methyldibenzofuran-3-ol, 6,8-Dichlor-7-methyl-dibenzofuran-3-ol, 6-Trifluormethyl-dibenzofuran-3-ol, 7-Trifluormethyl-dibenzofuran-3-ol, 7-tert.-Butyl-dibenzofuran-3-ol, 7-Methoxydibenzofuran-3-ol, 7-Chlor-2-nitro-dibenzofuran-3-ol und 7-Ethoxy-dibenzofuran-3-ol.

Die folgenden Beispiele erläutern die Herstellung der Verbindungen der Formel I.

<u>Beispiel 1</u>

a) Zu einer Lösung von 10,9 g (0,05 mol) 7-Chlor-dibenzofuran-3-ol in 30 ml trockenem Dimethylformamid werden 3,45 g (0,025 mol) fein gepulvertes Kaliumcarbonat zugegeben und anschließend bei 100°C in etwa 30 Minuten 10,2 g (0,1 mol) Propylencarbonat. Nach dreistündigem Rühren bei 120°C wird unter vermindertem Druck eingeengt, der Rückstand in Essigester aufgenommen und dreimal mit Wasser gewaschen. Nach Trocknen und Einengen der organischen Phase erhält man 13,3 g eines Isomerengemisches (ca. 2:1) von 1-(7'-Chlor-3'-dibenzofuranoxy)-propan-2-ol und 2-(7'-Chlor-3'-dibenzofuranoxy)-propan-1-ol. Schmp. 80 bis 82°C.

b) 45,2 g (0,163 mol) dieses Isomerengemisches werden, wie in Beispiel 2b) beschrieben, mit Thionylchlorid umgesetzt. Das dabei anfallende Rohprodukt wird in Petrolether gelöst. Beim Abkühlen kristallisieren 15,3 g 1-(7'-Chlor-3'-dibenzofuranoxy)-2-chlorpropan mit Schmp. 91 bis 92°C aus.

c) 15,3 g (0,05 mol) 1-(7'-Chlor-3'-dibenzofuranoxy)-2-chlorpropan und 25,5 g (0,3 mol) Piperidin werden bei 100°C 3 Tage gerührt, abgekühlt, in 250 ml Essigester aufgenommen und mit 50 ml 10 %iger (Gew.%) Natronlauge sowie zweimal mit Wasser gewaschen. Nach Trocknen und Einengen der organischen Phase löst man das verbliebene Öl in Ether, leitet bei 0°C Chlorwasserstoff ein, saugt ab und neutralisiert den Rückstand anschließend in Dichlormethan mit 5 %iger Natronlauge. Es wird mit Wasser neutral gewaschen, getrocknet und eingeengt. Dabei erhält man 8,1 g 1-(7'-Chlor-3'-dibenzofuranoxy)-2-piperidyl-propan mit Schmp. 71°C.

d) 8,0 g (0,023 mol) 1-(7'-Chlor-3'-dibenzofuranoxy)-2-piperidyl-propan gelöst in 40 ml trockenem Acetonitril und 40 ml trockenem Essigester wurden mit 0,9 ml einer 5 molaren Methylbromidlösung in Acetonitril (0,046 mol) versetzt und 24 Stunden bei Raumtemperatur (20°C) gerührt. Man saugte ab, wusch mit Acetonitril nach und erhielt nach Trocknen 3,5 g N-Methyl-N-2-(1-(7'-Chlor-3'-dibenzofuranoxy)-propyl)-piperidiniumbromid. Schmp. 185 bis 190°C (Verbindung Nr. 16).

Beispiel 2

a) Zu einer bei 0°C unter Inertgas gerührten Suspension von 30 g (0,78 mol) Lithiumaluminiumhydrid in 300 ml trockenem Tetrahydrofuran tropft man 164,5 g (0,54 mol) 2-(7'-Chlor-3'-dibenzofuranoxy)-propionsäuremethylester gelöst in 600 ml Tetrahydrofuran. Dabei steigt die Temperatur auf ca. 40°C. Man rührt ca. 3 Stunden bei 50°C nach, hydrolysiert nach Abkühlen auf 0°C mit 180 ml Wasser und neutralisiert mit Salzsäure. Es wird mit 2 l Methyl-tert.-butylether extrahiert, und nach Trocknen und Einengen der organischen Phase fallen 154,8 g 2-(7'-Chlor-3'-dibenzofuranoxy)-propan-1-ol an. Schmp. 65 bis 67°C.

b) Zu einer Suspension von 363,8 g (1,31 mol) 2-(7'-Chlor-3-dibenzofuranoxy)-propan-1-ol in 1,9 l trockenem Toluol werden 114 g (1,44 mol) Pyridin zugesetzt und anschließend bei maximal 20°C 119,7 ml (1,64 mol) Thionylchlorid zugetropft. Das Gemisch wird 2 Tage bei 50 bis 60°C gerührt, abgekühlt und nach Verdünnen mit 2,4 l Dichlormethan in 3,6 l Eiswasser eingerührt. Die organische Phase wird mit Wasser, 2 %iger Natronlauge und nochmals mit Wasser gewaschen, getrocknet und eingeengt. Man erhält 333,8 g 2-(7'-Chlor-3'-dibenzofuranoxy)-1-chlorpropan. Schmp. 62 bis 65°C. Die weitere Umsetzung erfolgt in gleicher Weise wie in Beispiel 1c und 1d beschrieben.

Entsprechend wurden diejenigen in der folgenden Tabelle angegebenen Verbindungen der Formel I hergestellt, deren Schmelzpunkte (FP) angegeben sind. Ihre Struktur wurde durch Ultrarot- und Kernresonanz-Spektroskopie oder durch Elementaranalyse gesichert. Die Verbindungen, bei denen keine Angaben zur physikalischen Chemie gemacht sind, können auf die gleiche Art und Weise wie bei den tatsächlich hergestellten Verbindungen erhalten werden; es kann erwartet werden, daß sie aufgrund ihrer ähnlichen Konstitution ähnliche Wirkungen wie die näher untersuchten Verbindungen haben.

| Verbindung Nr. | $R^1_n$ | W | p | q | A | Y | Fp [°C] |
|---|---|---|---|---|---|---|---|
| 1 | 7-Chlor | $CH(CH_3)CH_2$ | 0 | 0 | 1-Benzyl-piperidinium | Br | 142-145 |
| 2 | " | " | 0 | 0 | 1-Methyl-piperidinium | Br | 230-235 |
| 3 | " | " | 0 | 0 | 1-(2,4-Dichlorbenzyl)-piperidinium | Cl | 143-145 |
| 4 | " | " | 0 | 0 | 4-Benzyl-morpholinium | Br | 140-143 |
| 5 | " | " | 0 | 0 | 4-Allyl-morpholinium | Br | 240 (Zers.) |
| 6 | " | " | 0 | 0 | 4-Methyl-morpholinium | Br | 115 (Zers.) |
| 7 | " | " | 0 | 0 | 1-(4-tert.Butyl-benzyl)-piperidinium | Br | 205 (Zers.) |
| 8 | " | " | 0 | 0 | 4-tert.Butyl-1-crotyl-piperidinium | Br | 110-116 |
| 9 | " | " | 0 | 0 | 4-tert.Butyl-1-(4-Methylbenzyl)-<br>-piperidinium | Cl | 186-190 |
| 10 | " | " | 0 | 0 | 4-(4-Methylbenzyl)-2,6-dimethyl-<br>morpholinium (cis/trans-Isomeren-<br>gemisch) | Br | 106-111 |
| 11 | " | " | 0 | 0 | 4-Benzyl-2,6-dimethyl-morpholinium<br>(cis/trans-Isomerengemisch) | Cl | 105-110 |
| 12 | " | " | 0 | 0 | 2,4,6-Trimethylmorpholinium<br>(cis/trans-Isomerengemisch) | Br | 240 |
| 13 | 7-Methyl | " | 0 | 0 | 1-Methylpiperidinium | Br | 80- 90 |
| 14 | " | " | 0 | 0 | 1-(4-Chlorbenzyl)-piperidinium | Br | 178-184 |
| 15 | " | " | 0 | 0 | 1-(4-tert.Butylbenzyl)-piperidinium | Br | 203-206 |
| 16 | 7-Cl | $CH_2-CH(CH_3)$ | 0 | 0 | 1-Methylpiperidinium | Br | 185-190 |
| 17 | " | $CH(CH_3)CH_2$ | 0 | 0 | 4-(4-Methylbenzyl)-cis-2,6-dimethyl-<br>morpholinium | Br | 138-142 |
| 18 | " | " | 0 | 0 | 4-(4-Methylbenzyl)-trans-2,6-<br>-dimethylmorpholinium | Br | 115-125 |

| Verbindung Nr. | $R^1_n$ | W | p | q | A | Y | Fp [°C] |
|---|---|---|---|---|---|---|---|
| 19 | 7-Cl | $CH_2CH_2CH(CH_3)CH_2CH_2$ | 0 | 0 | 1-(4-tert.Butylbenzyl)-piperidinium | Br | 63-67 |
| 20 | " | $CH(C_2H_5)CH_2$ | 0 | 0 | 1-(4-tert.Butylbenzyl)-piperidinium | Br | |
| 21 | " | $CH(C_3H_7)CH_2$ | 0 | 0 | Trimethylammonium | Br | |
| 22 | " | $CH(CH_3)CH_2$ | 0 | 0 | Dimethyl-(4-fluorphenoxypropyl)-ammonium | Br | |
| 23 | " | " | 0 | 0 | 1-(4-Trifluormethylphenoxybutyl)--piperidinium | Br | |
| 24 | 7-Br | " | 0 | 0 | 4-(4-Chlorphenoxypentyl)-2,6--dimethylmorpholinium | Br | |

| Verbindung Nr. | $R^1_n$ | $R^3_q$ | W | p | A | Y | Fp [°C] |
|---|---|---|---|---|---|---|---|
| 25 | 6,7-$Cl_2$ | q=0 | $CH(CH_3)CH_2$ | 0 | 1-Methylpiperidinium | Br | |
| 26 | 7-Cl | 2-$NO_2$ | $CH(CH_3)CH_2$ | 0 | 1-(4-tert.Butylbenzyl)--piperidinium | Br | |
| 27 | 7-$OCH_3$ | q=0 | $CH(CH_3)CH_2$ | 0 | 4-(4-Chlorbenzyl)-morpholinium | Br | |

| Verbindung Nr. | $R^1_n$ | $R^2_p$ | W | q | A | Y | Fp [°C] |
|---|---|---|---|---|---|---|---|
| 28 | 6,8-$Cl_2$ | 7-$CH_3$ | $CH(CH_3)CH_2$ | 0 | 1-(4-Chlorbenzyl)-piperidinium | Br | |

Die neuen Wirkstoffe zeigen eine starke fungitoxische Wirksamkeit gegen phytopathogene Pilze. Sie dienen insbesondere zur Verhütung und Heilung von Pflanzenkrankheiten, die durch Mikroorganismen verursacht werden, wie z.B. Botrytis cinerea, Plasmopara viticola, Monilia fructigena, Alternaria solani, Sclerotinia sclerotiorum, Pyricularia oryzae, Pellicularia sasakii, Erysiphe graminis, Erysiphe cichoracearum, Chaetomium globosum, Aspergillus niger, Xanthomonas oryzae, Xanthomonas citri, Phytophthora infestans (an Kartoffeln und Tomaten).

Die neuen Wirkstoffe können gleichzeitig das Wachstum von zwei oder mehr der genannten Pilze unterdrücken und besitzen eine hohe Pflanzenverträglichkeit. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen.

Die fungiziden Mittel enthalten 0,1 bis 95 % (Gewichtsprozent) Wirkstoff, vorzugsweise 0,5 bis 90 %. Die Aufwandmengen liegen je nach Art des gewünschten Effektes zwischen 0,1 bis 5 kg Wirkstoff je ha.

Die neuen Wirkstoffe können auch zusammen mit anderen Wirkstoffen, z.B. Herbiziden, Insektiziden, Wachstumsregulatoren und anderen Fungiziden, oder auch mit Düngemitteln vermischt ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf, d.h. die fungizide Wirksamkeit des Kombinationsproduktes ist größer als die der addierten Wirksamkeiten der Einzelkomponenten. Eine besonders günstige Vergrößerung des Wirkungsspektrums wird mit folgenden Fungiziden erzielt:

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester

heterocyclische Strukturen, wie
2-Heptadecyl-2-imidazolin-acetat
2,4-Dichlor-6-(o-chloranilino)-s-triazin
O,O-Diethyl-phthalimidophosphonothioat
5-Amino-1-(bis-(dimethylamino)-phosphinyl)-3-phenyl1,2,4-triazol)
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethyl-phthalimid
N-Dichlorfluormethylthio-N',N'-dimethyl-N-phenylschwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin-4,4-dioxid
2,3-Dihydro-5-carboxanilido-6-methyl-1,4-oxathiin
2-Methyl-5,6-dihydro-4H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin

N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-
-triazol
1-[2-(2,4-Dichlorphenyl)-4-n-propyl-1,3-dioxolan-2-ylethyl]-1H-1,2,4-
-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-
α-(2-Chlorphenyl)-α-(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyethyl)-glutarimid
Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid
2-Cyano-N-(ethylaminocarbonyl)-2-methoximino-acetamid
2-(Thiocyanomethylthio)-benzthiazol
1-[2-(2,4-Dichlorphenyl)-4-ethyl-1,3-dioxolan-2-yl-methyl]-1H-1,2,4-
-triazol
2,4,5,6-Tetrachlor-isophthalodinitril
Methylenbisthiocyanat
Tributylzinnoxid
Mercaptobenzthiazol
Benzisothiazolon und seine Alkalisalze
Alkaliverbindungen des N'-Hydroxy-N-cyclohexyl-diazeniumoxids
2-(Methoxy-carbonylamino)-benzimidazol
2-Methyl-3-oxo-5-chlor-thiazolin-3-on
Trihydroxymethyl-nitro-methan
Glutardialdehyd
Chloracetamid

Die neuen Wirkstoffe werden beispielsweise in Form von direkt versprühbaren Lösungen, Suspensionen, Emulsionen, auch in Form von hochprozentigen wäßrigen, öligen oder sonstigen Dispersionen, Pasten, Stäubemitteln, Streumitteln, Granulaten durch Versprühen, Verstäuben, Verstreuen, Verstreichen oder Gießen ausgebracht. Die Anwendungsformen richten sich ganz nach den Verwendungszwecken; sie sollten in der Regel möglichst eine feine Verteilung der neuen Wirkstoffe gewährleisten.

Zur Herstellung von direkt oder nach Emulgieren in Wasser verwendbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische oder aromatische Kohlenwasserstoffe, zum Beispiel Benzol, Toluol, Xylol, Paraffin, Tetrahydronaphthalin, alkylierte Naphthaline oder deren Derivate z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw. in Betracht. Bevorzugt wird die Lösung in Wasser.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Phospolipide Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Beispiele für solche Pflanzenschutzmittel-Zubereitungen sind:

I. Man vermischt 90 Gewichtsteile der Verbindung des Beispiels 1 mit 100 Gewichtsteilen N-Methylpyrrolidon und erhält eine Lösung, die zur Anwendung in Form kleinster Tropfen geeignet ist.

II. 20 Gewichtsteile der Verbindung 3 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Doedecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung 5 werden in einer Mischung gelöst, die aus 30 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und Verrühren der Lösung in Wasser erhält man eine wäßrige Dispersion.

IV. 20 Gewichtsteile der Verbindung 8 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und Verrühren der Lösung in Wasser, erhält man eine wäßrige Dispersion.

V. 20 Gewichtsteile der Verbindung 9 werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in Wasser erhält man eine Spritzbrühe.

VI. 5 Gewichtsteile der Verbindung 10 werden mit 95 Gewichtsteilen feteiligem Kaolin innig vermischt. Man erhält auf diese Weise ein Stäubemittel, das 5 Gew.-% des Wirkstoffs enthält.

VII. 30 Gewichtsteile der Verbindung 11 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung 15 werden mit 30 Teilen Natriumsalz eines Phenolsulfonsäure-Harnstoff-Formaldehyd-Kondensates, 2 Teilen Kieselgel und 48 Teilen Wasser innig vermischt. Man erhält eine stabile wäßrige Dispersion.

IX. 20 Teile der Verbindung 3 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Auch für technische Zwecke im Materialschutz, z.B. als Holzschutzmittel, sind die neuen Ammoniumsalze geeignet. Über die fungizide Wirkung hinaus wurde auch eine bakterizide Wirksamkeit der Verbindungen festgestellt, so daß sie auch als solche im Pflanzenschutz und als technische Mikrozide in Betracht kommen. Beispielsweise können folgende Mikroorganismen damit bekämpft werden.

Staphylococcus aureus, Escherichia coli, Klebsiella pneumoniae, Citrobacter freundii, Proteus vulgaris, Pseudomonas aeruginosa, Pseudomonas fluorescens, Xanthomonas vesicatoria, Xanthomonas malvaccarum, Erwinia carotovora, Erwinia amylovora, Desulfovibrio desulfuricans, Streptoverticillium rubrireticuli, Aspergillus niger, Aspergillus versicolor, Penicillium funiculosum, Paecilomyces variotii, Trichoderma viride, Chaetomium globosum, Candida albicans, Geotrichum

candidans, Monilia sitophila, Scenedesmus quadricauda, Chlorella vulgaris, Nostoc muscorum.

Die üblichen Anwendungskonzentrationen betragen 0,01 bis 1 % Wirkstoff, bezogen auf das Gewicht des zu schützenden Materials; beim Einsatz zur Wasserbehandlung bei der Erdölförderung, in Schwimmbädern, Rückkühlwerken, Luftbefeuchtungsanlagen, Blumenfrischhaltemitteln oder in der Papierindustrie sind Wirkstoffmengen von 5 bis 100 ppm ausreichend, um eine Mikroorganismenentwicklung zu unterdrücken. Gebrauchsfertige Desinfektionsmittellösungen enthalten 0,2 bis 5 % Wirkstoff.

Die folgenden Versuche erläutern die biologische Wirkung der neuen Verbindungen. Vergleichsmittel (A) ist der bekannte Wirkstoff N-Trichlormethylthio-tetrahydrophthalimid (Chem. Week, June 21, 1972, Seite 46).

Versuch 1

Wirksamkeit gegen Phytophthora infestans an Tomaten

Blätter von Topfpflanzen der Sorte "Große Fleischtomate" werden mit wäßriger Spritzbrühe, die 80 % Wirkstoff und 20 % Emulgiermittel in der Trockensubstanz enthält, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Das Ergebnis des Versuches zeigt, daß beispielsweise die Verbindungen 1, 3, 5, 8, 9, 10, 14 und 15 bei der Anwendung als 0,025 %ige (Gew.%) Spritzbrühe eine bessere fungizide Wirkung (beispielsweise 90 %) zeigten als der bekannte Wirkstoff A (60 %).

Versuch 2

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so

stark entwickelt, daß die entstandenen Blattnekrosen den überwiegenden Teil der Blätter bedecken.

Das Ergebnis des Versuches zeigt, daß beispielsweise die Verbindungen 1, 2, 3, 4, 7, 8, 9, 10, 14, 15 und 16 bei der Anwendung als 0,05 %ige Spritzbrühe eine bessere fungizide Wirkung (beispielsweise 97 %) haben, als der bekannte Wirkstoff A (70 %).

Versuch 3

Zur Prüfung der Wirksamkeit gegenüber Pilzen werden die Wirkstoffe einer für das Wachstum der Pilze Aspergillus niger, Oidium lactis bzw. Candida albicans optimal geeigneten Nährlösung in Mengen von 100, 50, 25, 12, 6 und 3 Gewichtsteilen pro Million Teile Nährlösung zugesetzt. Jeweils 10 ml Nährlösungs-Wirkstoffgemisch werden in sterile Reagenzgläser gegeben und mit einem Tropfen einer Sporensuspension beimpft, die $10^6$ Konidien bzw. Zellen enthält. Nach 120stündiger Bebrütung wird die Verdünnungsstufe ermittel, bei welcher kein sichtbares Wachstum der Pilze mehr erfolgt; sie wird in der Tabelle als minimale Hemmstoffkonzentration angegeben.

Das Ergebnis des Versuches zeigt, daß beispielsweise die Verbindungen 14, 15 und 16 eine gute fungizide Wirkung haben (beispielsweise 12 Teile Wirkstoff pro 1 Million Teile Nährlösung).

Versuch 4

Zur Ermittlung der Wirksamkeit der neuen Verbindungen gegenüber Bakterien werden zu je 5 ml steigender Verdünnung der Wirkstoffe 5 ml doppelt konzentrierter Nährbouillon in sterile Reagenzgläser gegeben und vermischt. Durch Zugabe von einem Tropfen einer 1:10 verdünnten 16 Stunden alten Bouillon-Kultur der Bakterien-Arten Staphylococcus aureus bzw. Escherichia coli oder Pseudomonas aeruginosa werden dann die Röhrchen beimpft und 24 Stunden bei 37°C bebrütet. Nach dieser Zeit werden Proben aus den Röhrchen auf Bakteriennährböden übertragen und diese ebenfalls 24 Stunden lang bei 37°C bebrütet. Diejenige Verdünnungsstufe, bei welcher keine sichtbare Bakterienentwicklung mehr erfolgt, wird als minimale Hemmkonzentration angegeben.

Das Ergebnis des Versuches zeigt, daß beispielsweise die Verbindungen 14, 15 und 16 eine gute bakterizide Wirkung haben (beispielsweise 12 Teile Wirkstoff pro 1 Million Teile Nährlösung).

Patentansprüche

1. Quartäres Ammoniumsalz der Formel

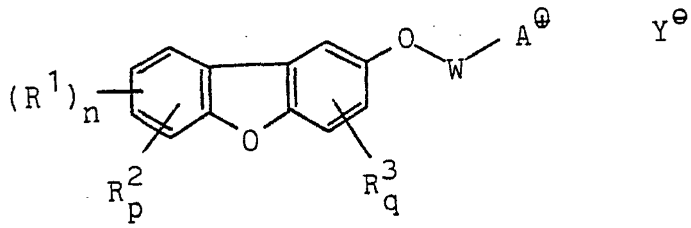

(I)

in der $R^1$, $R^2$ und $R^3$ gleich oder verschieden sind und Halogen, eine gegebenenfalls halogensubstituierte Alkyl- oder Alkoxygruppe mit 1 bis 4 Kohlenstoffatomen, Cyan oder Nitro,
n, p und q die Zahlen 0, 1, 2 oder 3,
W eine durch eine oder mehrere Alkylgruppen mit jeweils 1 bis 3 C-Atomen substituierte Alkylenkette mit 2 bis 5 C-Atomen,
A die Gruppe $-NR^4R^5R^6$, in der $R^4$, $R^5$ und $R^6$ gleich oder verschieden sind und unabhängig voneinander einen Alkyl-, Alkenyl- oder Alkinylrest jeweils mit bis zu 6 Kohlenstoffatomen, einen Cycloalkylrest mit 3 bis 12 Kohlenstoffatomen oder einen Cycloalkenylrest mit 4 bis 7 Kohlenstoffatomen, wobei diese acyclischen und cyclischen Reste durch Halogen, Cyan, Alkoxy mit 1 bis 4 Kohlenstoffatomen oder Dialkylamino mit 1 bis 4 Kohlenstoffatomen in jeder Alkylgruppe substituiert sein können, oder einen gegebenenfalls durch Fluor, Chlor, Brom, Alkyl-, Alkenyl- oder Alkoxyrest jeweils mit bis zu 4 Kohlenstoffatomen, Trifluormethyl, Nitro, Cyan oder Alkoxycarbonyl mit 2 bis 5 Kohlenstoffatomen substituierten Aralkyl- oder Aryloxyalkylrest bedeuten oder $R^5$ zusammen mit $R^6$ Teil eines gegebenenfalls durch eine oder mehrere Alkylgruppen mit 1 bis 4 Kohlenstoffatomen substituierten 5-, 6- oder 7-gliedrigen heterocyclischen Ringes mit 1 bis 3 Heteroatomen ist, und
Y das Anion einer nicht phytotoxischen Säure HY bedeutet.

2. Fungizides Mittel, enthaltend ein Dibenzofuranderivat der Formel I gemäß Anspruch 1.

3. Fungizides Mittel, enthaltend inerte Zusatzstoffe und ein Dibenzofuranderivat der Formel I gemäß Anspruch 1.

4. Verfahren zur Bekämpfung von Pilzen, dadurch gekennzeichnet, daß man ein Dibenzofuranderivat der Formel I gemäß Anspruch 1 auf die

Pilze oder auf durch Pilzbefall bedrohte Materialien, Flächen, Pflanzen oder Saatgüter einwirken läßt.

5. Verfahren zur Herstellung eines fungiziden Mittels, dadurch gekennzeichnet, daß man ein Dibenzofuranderivat der Formel I gemäß Anspruch 1 mit einem festen oder flüssigen Trägerstoff sowie gegebenenfalls mit einem oder mehreren oberflächenaktiven Mitteln mischt.

6. Verfahren zur Herstellung von Dibenzofuranderivaten der Formel I gemäß Anspruch 1, dadurch gekennzeichnet, daß man

a) eine Verbindung der Formel

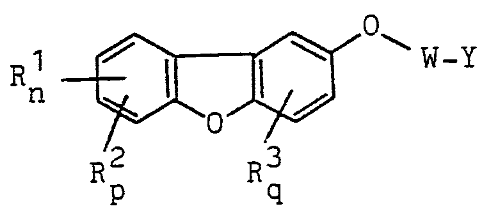

(II)

in der $R^1$, $R^2$, $R^3$, W, Y, n, p und q die im Anspruch 1 angegebenen Bedeutungen haben, mit einem tertiären Amin der Formel A entsprechend den in Anspruch 1 angegebenen Bedeutungen oder

b) eine Verbindung der Formel

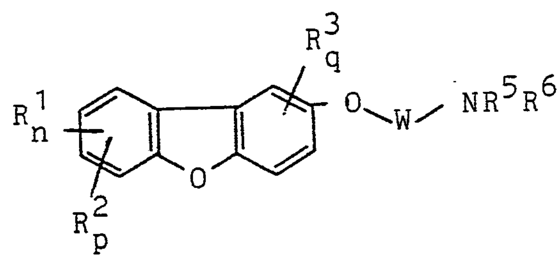

(III),

in der $R^1$, $R^2$, $R^3$, $R^5$, $R^6$, W, n, p und q die im Anspruch 1 angegebenen Bedeutungen haben, mit einer Verbindung der Formel

$$R^4 - Y \qquad (IV),$$

in der $R^4$ und Y die im Anspruch 1 angegebenen Bedeutungen haben, umsetzt.

Patentansprüche

1. Fungizides Mittel, enthaltend ein quartäres Ammoniumsalz der Formel

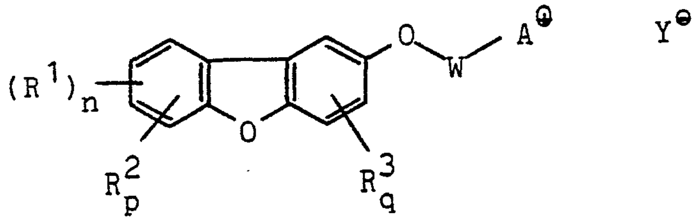

(I)

in der $R^1$, $R^2$ und $R^3$ gleich oder verschieden sind und Halogen, eine gegebenenfalls halogensubstituierte Alkyl- oder Alkoxygruppe mit 1 bis 4 Kohlenstoffatomen, Cyan oder Nitro, n, p und q die Zahlen 0, 1, 2 oder 3, W eine durch eine oder mehrere Alkylgruppen mit jeweils 1 bis 3 C-Atomen substituierte Alkylenkette mit 2 bis 5 C-Atomen, A die Gruppe $-NR^4R^5R^6$, in der $R^4$, $R^5$ und $R^6$ gleich oder verschieden sind und unabhängig voneinander einen Alkyl-, Alkenyl- oder Alkinylrest jeweils mit bis zu 6 Kohlenstoffatomen, einen Cycloalkylrest mit 3 bis 12 Kohlenstoffatomen oder einen Cycloalkenylrest mit 4 bis 7 Kohlenstoffatomen, wobei diese acyclischen und cyclischen Reste durch Halogen, Cyan, Alkoxy mit 1 bis 4 Kohlenstoffatomen oder Dialkylamino mit 1 bis 4 Kohlenstoffatomen in jeder Alkylgruppe substituiert sein können, oder einen gegebenenfalls durch Fluor, Chlor, Brom, Alkyl-, Alkenyl- oder Alkoxyrest jeweils mit bis zu 4 Kohlenstoffatomen, Trifluormethyl, Nitro, Cyan oder Alkoxycarbonyl mit 2 bis 5 Kohlenstoffatomen substituierten Aralkyl- oder Aryloxyalkylrest bedeuten oder $R^5$ zusammen mit $R^6$ Teil eines gegebenenfalls durch eine oder mehrere Alkylgruppen mit 1 bis 4 Kohlenstoffatomen substituierten 5-, 6- oder 7-gliedrigen heterocyclischen Ringes mit 1 bis 3 Heteroatomen ist, und Y das Anion einer nicht phytotoxischen Säure HY bedeutet.

2. Fungizides Mittel, enthaltend inerte Zusatzstoffe und ein Dibenzofuranderivat der Formel I gemäß Anspruch 1.

3. Verfahren zur Bekämpfung von Pilzen, dadurch gekennzeichnet, daß man ein Dibenzofuranderivat der Formel I gemäß Anspruch 1 auf die Pilze oder auf durch Pilzbefall bedrohte Materialien, Flächen, Pflanzen oder Saatgüter einwirken läßt.

4. Verfahren zur Herstellung eines fungiziden Mittels, dadurch gekennzeichnet, daß man ein Dibenzofuranderivat der Formel I gemäß Anspruch 1 mit einem festen oder flüssigen Trägerstoff sowie gegebenenfalls mit einem oder mehreren oberflächenaktiven Mitteln mischt.

5. Verfahren zur Herstellung von Dibenzofuranderivaten der Formel I gemäß Anspruch 1, dadurch gekennzeichnet, daß man

a) eine Verbindung der Formel

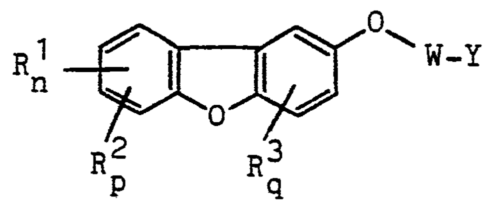

(II)

in der $R^1$, $R^2$, $R^3$, W, Y, n, p und q die im Anspruch 1 angegebenen Bedeutungen haben, mit einem tertiären Amin der Formel A entsprechend den in Anspruch 1 angegebenen Bedeutungen oder

b) eine Verbindung der Formel

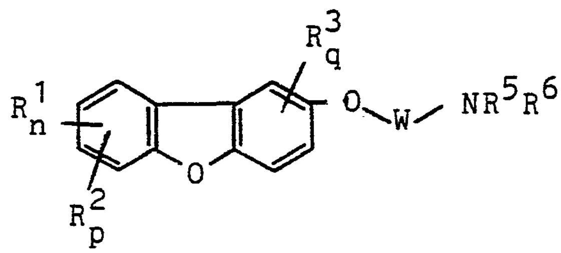

(III),

in der $R^1$, $R^2$, $R^3$, $R^5$, $R^6$, W, n, p und q die im Anspruch 1 angegebenen Bedeutungen haben, mit einer Verbindung der Formel

$$R^4 - Y \qquad (IV),$$

in der $R^4$ und Y die im Anspruch 1 angegebenen Bedeutungen haben, umsetzt.

Europäisches Patentamt

**EUROPÄISCHER RECHERCHENBERICHT**

0103277

Nummer der Anmeldung

EP 83 10 8871

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| X | EP-A-0 057 362 (BASF) *Insgesamt* ----- | 1-6 | C 07 D 307/91<br>C 07 D 405/12<br>C 07 D 413/12<br>A 01 N 43/12 |
| | | | RECHERCHIERTE SACHGEBIETE (Int. Cl. 3) |
| | | | C 07 D 405/00<br>C 07 D 413/00<br>C 07 D 307/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 06-12-1983 | ALLARD M.S. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : *technologischer Hintergrund*
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument